Europäisches Patentamt

European Patent Office

Office européen des brevets

(11) Publication number: **0 073 183**
**B1**

(12) **EUROPEAN PATENT SPECIFICATION**

(45) Date of publication of patent specification: **17.04.85**

(21) Application number: **82850171.8**

(22) Date of filing: **17.08.82**

(51) Int. Cl.⁴: **A 41 B 9/00,** A 61 F 13/16,
A 41 B 13/04

(54) **Elastic pants.**

(30) Priority: **26.08.81 SE 8105061**

(43) Date of publication of application:
**02.03.83 Bulletin 83/09**

(45) Publication of the grant of the patent:
**17.04.85 Bulletin 85/16**

(84) Designated Contracting States:
**BE DE FR GB NL**

(56) References cited:
**FR-A-1 117 957**
**FR-A-2 184 543**
**FR-A-2 241 645**
**GB-A-1 040 053**
**US-A-3 599 640**

(73) Proprietor: **Mölnlycke AB**
**S-405 03 Göteborg (SE)**

(72) Inventor: **Birring, Mats Gustav**
**Södra Viktoriagatan 27**
**S-411 25 Göteborg (SE)**

(74) Representative: **Alfredson, Stig et al**
**H. ALBIHNS PATENTBYRA AB Box 7664**
**S-103 94 Stockholm (SE)**

EP 0 073 183 B1

Courier Press, Leamington Spa, England.

## Description

The present invention relates to elastic pants or briefs made of an elastic material and designed for holding a disposable diaper in place and consisting of a front and a back knitted or crocheted piece, which are joined to each other along their side edges and at the crotch portion of the pants.

The now common disposable diapers with tape for holding in place do not provide sufficient security against leakage. This is primarily because there are too few points of attachment, whereby gaps easily occur between the semi-stiff diaper and the body of the user. The problem is especially pronounced with respect to incontinent adults, who of course want to be able to get about without the risk of urine leakage for example.

The use of separate pants or briefs for holding a diaper in place is of course greatly superior to diapers with tape as regards security against the diaper coming loose or sliding out of position. Despite this, the fact that most of the known briefs or pants for diapers are equally elastic everywhere, with the possible exception of the waist and leg edge portions, presents particular and hitherto unsolved leakage problems. Due to the different shapes of the human body and the diaper, the pressure exerted by the known briefs or pants will be greatest along the side of the user and at the middle of the diaper. The fixing of the diaper will thus be poor and the pressure against the side edges of the diaper will be minimal, resulting in the risk of leakage particularly at the edges.

Although pants which can hold, for example, medical aids, auxiliaries, sanitary and hygiene articles etc., and which have zones of differing elasticity are known from FR—A—2 241 645, the elasticity of the known garment is strongest in those regions opposite the article to be supported. Consequently, any liquid taken up thereby will be squeezed therefrom as the wearer moves, instead of being retained therein. The same disadvantage is found with the disposable undergarment and absorption pad disclosed in US—A—3 599 640, according to which the elastic in the garment presses against the absorption pad, such as to create an immediate risk of leakage. Thus, no completely satisfactory aid for incontinent people has been available.

Since incontinence is common and quite a problem especially for significant numbers of elderly people, the facts discussed here involve a social problem of major proportions. A number of studies have shown that many people become socially isolated due to incontinence. Many do not even venture outside the home to make necessary purchases for fear of urinating. For the same reasons, all normal social intercourse is ruled out.

In short, these social problems are a result of the fact that incontinent adult have, up to now, had to live with the feeling that they cannot depend on the aids used to provide satisfactory protection.

The present invention is the first to provide a solution to these problems which is satisfactory in all respects.

The invention has achieved a pair of elastic pants, which holds the diaper in place and increases the pressure against its edges. The major feature of the pants according to the invention is that the knitting or crocheting is looser in a central area in the front and back pieces, said area extending from the crotch portion to a location spaced from the waist edge of the pants, that elastic threads are knitted in or crocheted in to define a central portion of the front and back pieces, whereby, when the pants are used to hold a diaper in place, the limiting edges of the looser knitted or crocheted areas and the elastic threads press substantially against the edges of the diaper and provide an effective seal against leakage, and that the elastic threads on the respective pieces consist of a first thread or a first bundle of threads, which extends a distance from each side edge along the waist edge portion of the piece and thereafter a distance diagonally inwards towards the crotch portion to finally extend across the central portion of the pants parallel to the waist edge, and a second thread or a second bundle of threads, which extends from the respective side edge parallel with said waist edge portion towards the diagonally inwards extending part of the first thread or bundle of threads and thereafter converging towards the crotch portion parallel with the direction of said diagonally inwards extending first thread and to finally extend across the central portion of the pants parallel to the waist edge, whereby the several converging elastic threads cooperate to give tension against the periphery of a generally V-shaped central portion defined by the converging threads on each piece.

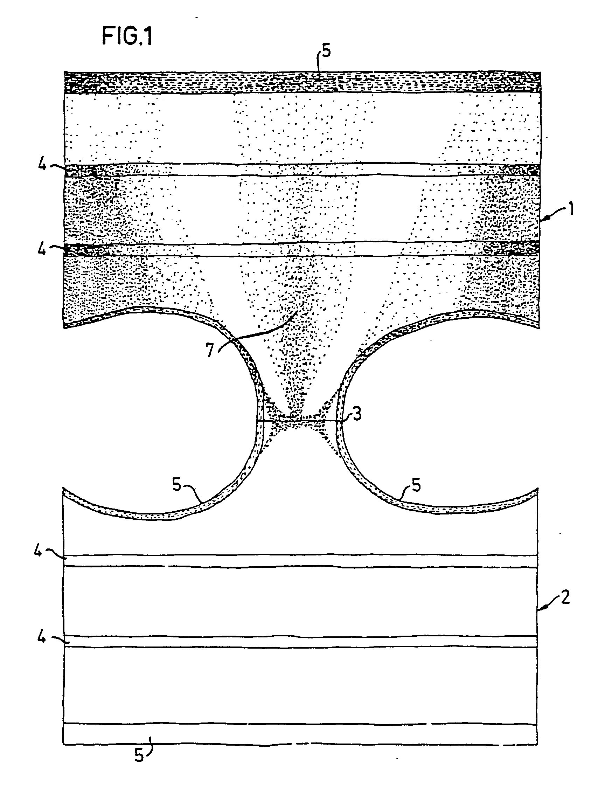

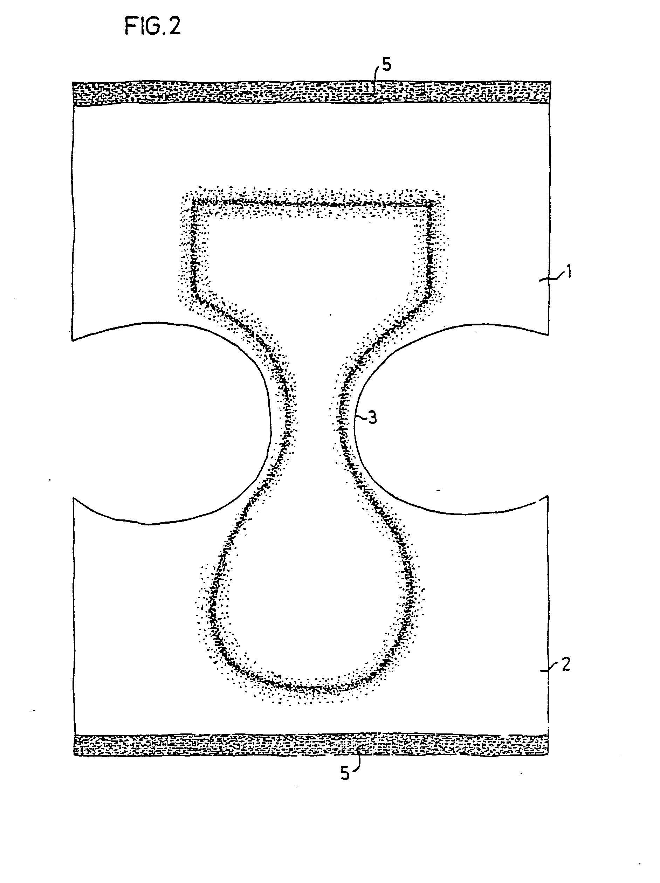

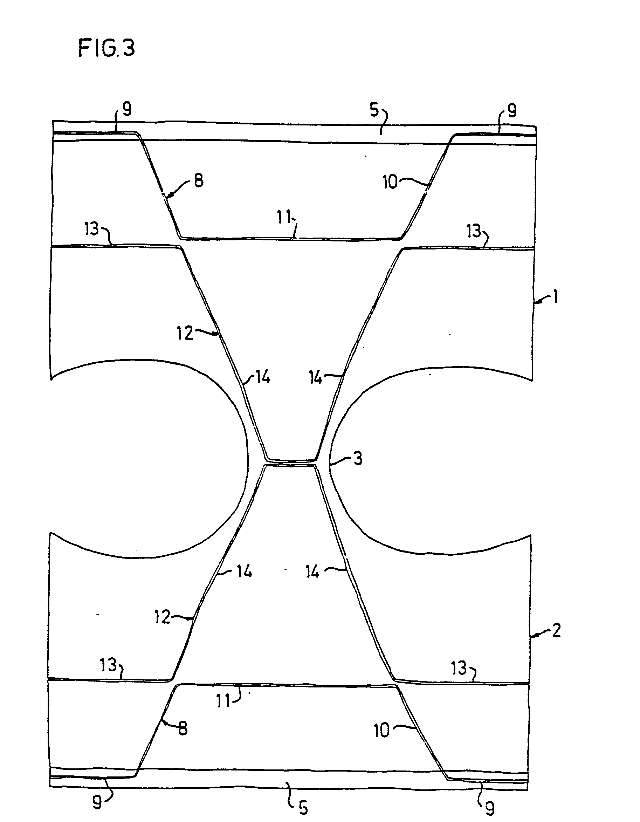

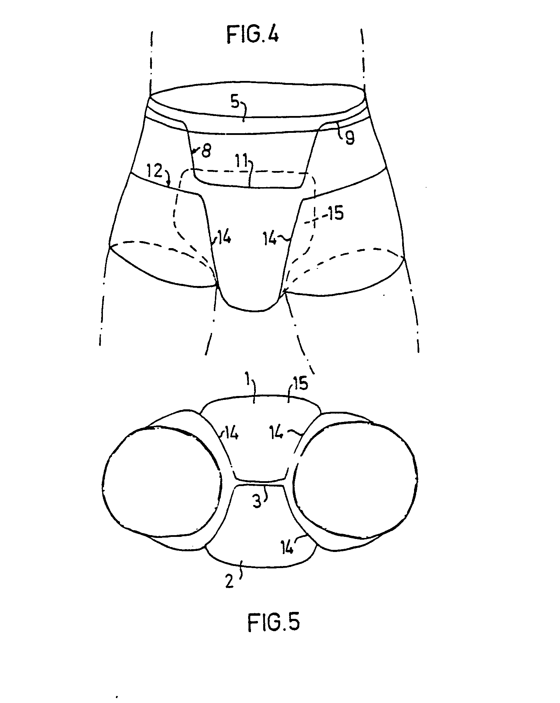

The invention will now be explained in the following with reference to examples, which are shown in the accompanying drawings. Fig. 1 illustrates the distribution of the pressure which previously known elastic pants exert against the underlying disposable diaper and the user. Fig. 2 shows the desired ideal pressure distribution. Fig. 3 shows a first embodiment of a pair of diaper pants according to the invention. Figs. 4 and 5 show the pants according to Fig. 3 when used to hold a disposable diaper in place.

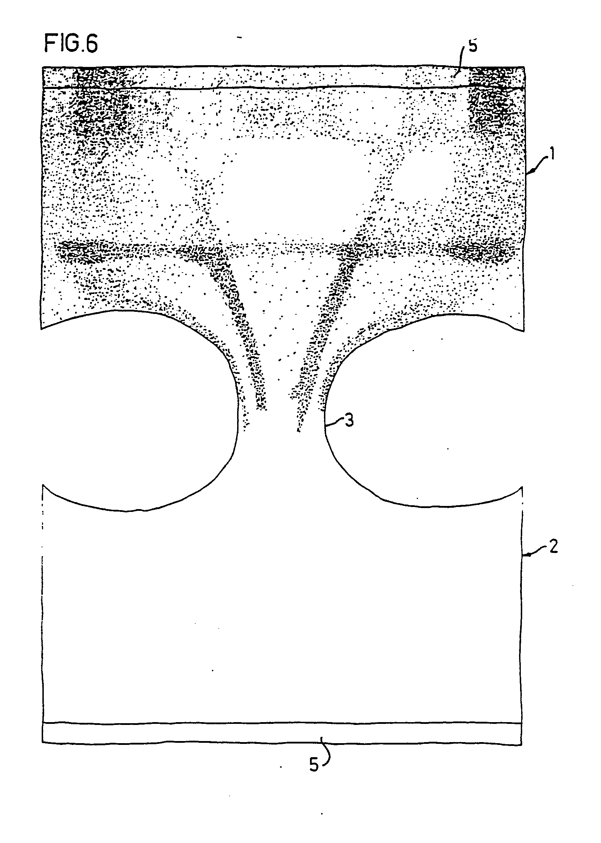

Fig. 6 shows the pressure exerted by the diaper according to Figs. 3—5 against the underlying disposable diaper and the user.

Fig. 1 shows the blank of a pair of elastic pants or briefs of previously known type consisting of a front and a back piece 1, 2, which are entirely identical and designed to be joined to each other along their side edges and at the crotch portion 3. The pants consist of crocheted pieces of an elastic material. Threads 4 of spandex are crocheted into the pants, but except for these and the waist gands 5 and the leg bands 6, the pants have the same elasticity everywhere. After putting the pants on over a disposable diaper, the pressure distribution illustrated in Fig. 1 is produced, where the darkest areas indicate the areas of greatest pressure.

It is easy to see that the pressure distribution resulting from the pants in Fig. 1 on an underlying diaper are entirely unsuitable. The pressure is greatest at 7, i.e. against the middle of the underlying disposable diaper, while it is least and almost non-existent at the light V-shaped area in the Figure, which covers the edge portions of the underlying diaper. Thus when the pants according to Fig. 1 are used, no edge pressure whatsoever is produced against the underlying diaper, which means that the risk of edge leakage will be especially great. Since the pressure is substantially exerted against the center of the disposable diaper, its position will not be stable and it can easily slide to one side. Furthermore, it is apparent that the pants provide poor containment of excrement, and when used by incontinent males, it also provides poor positioning of the penis which also substantially increases the risk of leakage.

The present invention, however, is based on the insight that the pressure exerted by the elastic pants or briefs against an underlying disposable diaper shown have a distribution of essentially the appearance shown in Fig. 2. This pressure distribution holds the diaper firmly in the correct position, provides increased pressure along the edges of the diaper and reduced pressure across the central portions, thus together providing effective enclosure of both urine and excrement within the area covered by the disposable diaper.

The elastic diaper according to the invention shown in Fig. 3 consists of a front and a back piece 1 and 2, respectively, of crocheted elastic material. These pieces are essentially evenly crocheted over their entire surfaces. The two pieces 1, 2 are thus entirely identical. For the purpose of providing a pressure distribution of at least essentially the type shown in Fig. 2 against an underlying disposable diaper, a number of elastic threads have been crocheted into each piece, for example of spandex yarn. The elastic threads in each piece consist of a first thread 8, which extends a distance 9 from each side edge along the waist edge portion and thereafter a distance 10 diagonally inwards towards the crotch portion 3 to finally extend over a portion 11 across the central portion of the pants parallel to the waist edge; and of a second thread 12 which extends with portions 13 from the respective side edge level with the central portion 11 of the first thread 8 up to said thread and thereafter with portions 14 converging towards the crotch portion.

Fig. 4 shows the appearance of the pants according to Fig. 3 when used for supporting an essentially T-shaped disposable diaper 15. The central portion 11 of the first elastic thread and the portions 14 of the second elastic thread converging towards the crotch portion 3 press the side edge portions of the diaper and a horizontal portion in the vicinity of the upper end of the diaper into tight contact against the body of the user. The back of the diaper is also tensioned correspondingly. Fig. 5 shows in a view from below how the edge portions of the disposable diaper 15 are pressed against the user. The pressure distribution caused by the pants according to Fig. 3 against the underlying disposable diaper and against the user is shown in Fig. 6. A comparison of the pressure distributions according to Figs. 1 and 2 shows that the pressure distribution caused by the elastic threads in the embodiment shown in Fig. 3 of a pair of pants according to the invention is nearly ideal.

The invention is not, however, limited to the embodiments described above. Rather, a number of modifications are possible within the scope of the following claims. The elastic threads need not of course be arranged exactly as described in connection with Fig. 3. What is important is that they produce a pressure distribution against the underlying disposable diaper of at least essentially the appearance in Figs. 2 and 6.

The elastic pants according to the invention have been described here in connection with the use of diapers by incontinent adults. The pants in question are, however, of course also suitable for children.

### Claim

Elastic pants or briefs made of an elastic material and intended for holding a disposable diaper (15) in place and consisting of a front (1) and a back (2) knitted or crocheted piece, which are joined to each other along their side edges and at the crotch portion (3) of the pants, characterized in that the knitting/crocheting is looser in a central area in the front (1) and back (2) pieces, said area extending from the crotch portion (3) to a location spaced from the waist edge of the pants, that elastic threads (8, 12) are knitted in or crocheted in to define a central portion of the front and back pieces, whereby, when the pants are used to hold a diaper in place, the limiting edges of the looser knitted or crocheted areas and the elastic threads (8, 12) press substantially against the edges of the diaper and provide an effective seal against leakage, and that the elastic threads (8, 12) on the respective pieces (1, 2) consist of a first thread (8) or a first bundle of threads, which extends a distance (9) from each side edge along the waist edge portion (5) of the piece and thereafter a distance (10) diagonally inwards towards the crotch portion (3) to finally extend across the central portion of the pants parallel to the waist edge, and a second thread (12) or a second bundle of threads, which extends from the respective side edge parallel with said waist edge portion towards the diagonally inwards extending part of the first thread or bundle of threads and thereafter converging towards the crotch portion parallel with the direction of said diagonally inwards extending first thread and to finally extend across the central portion of the pants parallel to the waist edge, whereby the several converging elastic threads cooperate to give tension against the periphery of a generally V-shaped central portion defined by the converging threads on each piece.

## Patentanspruch

Elastische Hosen oder Schlüpfer aus elastischem Material und bestimmt zum Halten einer Wegwerfeinlage (15) am Ort, bestehend aus einem gestrickten oder gehäkelten Vorderteil (1) und Hinterteil (2), die entlang ihrer Seitenränder und im Schrittabschnitt (3) der Hosen miteinander verbunden sind, dadurch gekennzeichnet, daß das Stricken/Häkeln in einem Mittelbereich des Vorderteils und des Hinterteils (2) loser ist, daß dieser Bereich vom Schritt (3) zu einer Stelle verläuft, die vom Taillenrand der Hosen beabstandet ist, daß elastische Fäden (8, 12) eingestrickt oder eingehäkelt sind, um einen Mittelabschnitt des Vorderteils und des Hinterteils zu bilden, wodurch, wenn die Hosen für das am Orthalten der Einlage verwendet werden, die begrenzenden Ränder der loser gestrickten oder gehäkelten Bereiche und die elastischen Fäden (8, 12) im wesentlichen gegen die Ränder der Einlage drücken und eine wirksame Abdichtung gegen eine Leckage vorsehen, und daß die elastischen Fäden (8, 12) auf den jeweiligen Teilen (1, 2) aus einem ersten Faden (8) oder einem ersten Bündel von Fäden bestehen, der oder das in einem Abstand (9) von jedem Seitenrand entlang dem Taillenrandabschnitt (5) des Stücks und danach in einem Abstand (10) diagonal nach innen in Richtung auf den Schrittabschnitt (3) verläuft, um letztlich quer über den Mittelabschnitt der Hosen parallel zum Taillenrand zu verlaufen, und aus einem zweiten Faden (12) oder einem zweiten Bündel von Fäden besteht, der oder das vom jeweiligen Seitenrand parallel zum Taillenrandabschnitt auf den diagonal nach innen verlaufenden Teil des ersten Fadens oder Bündels von Fäden verläuft und danach zum Schrittabschnitt parallel zur Richtung des diagonal nach innen verlaufenden ersten Fadens konvergiert und um schließlich quer über den Mittelabschnitt der Hosen parallel zum Taillenrand zu verlaufen, wodurch mehrere konvergierende elastische Fäden zusammenwirken, um gegen den Umfang eines hauptsächlich V-förmigen Mittelabschnittes, welcher durch die konvergierenden Fäden jedes Stücks gebildet wird, eine Spannung vorzusehen.

## Revendication

Slip ou culotte faite d'une matière élastique et destinée à tenir une couche à jeter ou non-réutilisable (15) en place et qui se compose d'un devant (1) et d'une partie arrière (2) tricotés ou faits au crochet, qui sont reliés l'un à l'autre le long de leurs bords latéraux et à la fourche (3) du slip, caractérisé en ce que le tissu tricoté ou crocheté est moins serré dans la région centrale du devant (1) et de la partie arrière (2), ladite région s'étendant de la fourchë (3) jusqu'à un emplacement espacé du bord de la ceinture du slip, en ce que des fils élastiques (8, 12) sont incorporés dans le tissu tricoté ou crocheté afin de délimiter une partie centrale du devant et de la partie arrière, ce qui fait que quand le slip est utilisé pour tenir une couche en place, les bords limitant les régions moins serrées du tissu tricoté ou crocheté et les fils élastiques (8, 12) exercent une pression contre les bords de la couche en produisant effectivement un joint étanche empêchant les fuites, et en ce les fils élastiques (8, 12) des parties respectives du slip (1, 2) comprennent un premier fil (8) ou un premier faisceau de fils qui s'étendent sur une certaine distance (9) de chaque bord latéral le long du bord de la ceinture (5) de la partie considérée et, de là, sur une certaine distance (10) en diagonale vers l'intérieur en direction de la fourche (3) pour s'étendre, finalement, le long de la partie centrale du slip parallèlement au bord de la ceinture, et un second fil (12) ou un second faisceau de fils qui s'étend des bords latéraux respectifs, parallèlement audit bord de ceinture, en direction de la partie s'étendant en diagonale vers l'intérieur du premier fil ou faisceau de fils, en convergent ensuite vers la fourche, parallèlement à la direction dudit premier fil s'étendant en diagonale vers l'intérieur, pour finalement s'étendre le long de la partie centrale du slip, parallèlement au bord de la ceinture, ce qui fait que les divers fils élastiques convergents coopèrent pour exercer une certaine tension contre le pourtour d'une partie centrale ayant une forme générale en V définie par les fils convergents de chaque partie du slip.

FIG.1

## FIG.2

FIG.3

FIG.4

FIG.5

FIG.6

5